# EUROPEAN PATENT APPLICATION

(11) **EP 4 417 701 A1**
(43) Date of publication of application: **21.08.2024**
(21) Application number: 22717334.1
(22) Date of filing: 24.02.2022
(51) Int. Cl.: C12Q 1/52, C12Q 1/32, C12Q 1/61, C12Q 1/44, C12Q 1/26, C12Q 1/48, C12Q 1/28

(54) **IN-VITRO DIAGNOSTIC REAGENT PRESERVATIVE AND USE THEREOF**

(30) Priority: 12.10.2021 CN 202111187518
(71) Applicant: Daan Gene Co., Ltd., Guangzhou, Guangdong 510665 (CN)
(72) Inventor: JIANG, Xiwen, Guangzhou, Guangdong 510665 (CN); QI, Wenchuang, Guangzhou, Guangdong 510665 (CN); WU, Runfeng, Guangzhou, Guangdong 510665 (CN); DUAN, Shaoqing, Guangzhou, Guangdong 510665 (CN); JIAN, Junxing, Guangzhou, Guangdong 510665 (CN); DIAO, Xue, Guangzhou, Guangdong 510665 (CN)
(74) Representative: Handsome I.P. Ltd
(86) International application number: PCT/CN2022/077614
(87) International publication number: WO 2023/060818

(57) **Abstract**

The embodiments of the present application, belonging to the technical field of medical test and assay, and provide a preservative for an in vitro diagnostic reagent. The preservative includes a combination of a sulfadoxine solution and a dimethoprim solution, wherein a molar ratio of sulfadoxine in the sulfadoxine solution to dimethoprim in the dimethoprim solution is from 0.002 to 1. The present disclosure also provides use of the preservative for the in vitro diagnostic reagent. The technical solutions according to the embodiments of the present disclosure improve stability of the reagent, do not affect reactions of the reagent, and may be extensively applied.

## Description

This application is based upon and claims priority to Chinese Patent Application No. 202111187518.7, filed before China National Intellectual Property Administration on October 12, 2021 and entitled "PRESERVATIVE FOR IN VITRO DIAGNOSTIC REAGENT AND USE THEREOF," the entire contents of which are incorporated herein by reference.

### TECHNICAL FIELD

The present disclosure relates to the technical field of medical test and assay, and in particular, relates to a preservative for an in vitro diagnostic reagent and use thereof.

### BACKGROUND

In vitro diagnostic reagents generally use water as a carrier and contain enzymes, antibodies, protein stabilizers, nucleic acids, polysaccharides, or the like. These contents are easy to breed microorganisms in the environment containing more water. Microorganisms may originate from airborne bacteria in the air or from bacteria brought by the raw materials. Upon entering a reagent, the microorganisms may grow exponentially in the reagent due to the influence of temperature and humidity. The production of a large number of microorganisms causes a decrease in the activity of the above-mentioned enzyme, antibody and other components, and indicators, such as assay precision, tend to deteriorate.

Preservatives currently used in in vitro diagnostic reagents generally include sodium azide, thiomersal, 5-bromo-5-nitro-1, 3-dioxane, imidazolidinyl urea, methyl isothiazolinone, and the like. Sodium azide is one of the most commonly used preservatives in in vitro diagnostic reagents. It can block the electron transport chain of cells and is toxic to many organisms. Sodium azide is similar to cyanide, belonging to highly toxic compounds, and achieves an inhibitory effect on cytochrome oxidase and other enzymes. In addition to causing irritations eyes and skin, sodium azide can also be inhaled, orally administered or absorbed by skin to cause toxic death. In addition, sodium azide may cause explosion when exposed to high heat or severe shock. For safety reasons, the use of sodium azide is increasingly limited.

Thimerosal has been used as a preservative for many years and has been widely used in the preservation of biological products. Thiomersal has strong antibacterial activity against Gram-positive and Gram-negative bacteria, and it is a broad-spectrum antibacterial agent. Its action mechanism is that heavy metal ions are bound to sulfhydryl groups of enzyme protein in bacteria to make the enzyme inactive. Thiomersal is a mercury-containing chemical substance with a mercury content accounting for 50% of the molecular mass, and the metabolic or degradation products are ethyl mercury and thiosalicylate, and ethyl mercury is an organic mercury degraded by dimethyl mercury. Thimerosal is no longer widely used as a preservative for safety reasons due to the theoretical neurotoxicity of small amounts of organic mercury.

In recent years, new antiseptics, such as 5-bromo-5-nitro-1,3-dioxane, imidazolidinyl urea, and methyl isothiazolinone, have attracted much attention. They are highly effective and exert a strong inhibitory effect on various bacteria, molds, yeasts, and algae. The mechanism is that they act on -COOH, -NH2, -OH, and-SH groups of cell proteins, resulting in partial inactivation of enzymes needed for growth and inhibition of microbial growth. In conventional diagnostic reagents, various tool enzymes are often added for the reaction, such as lactate dehydrogenase, and urease. Preservatives such as 5-bromo-5-nitro-1, 3-dioxane, imidazolidinyl urea, and methylisothiazolinone have been shown to inhibit a variety of tool enzymes. Therefore, these preservatives are not suitable for use in combination with the tool enzymes described above.

### SUMMARY

The technical problem to be solved by embodiments of the present application is that the preservative in the related art has toxicity, and inhibits the tool enzyme in the diagnostic reagent, resulting in a decrease in the activity of the tool enzyme, antibody, and the like, thereby resulting in a decrease in the assay precision of the diagnostic reagent.

In view of the above, the embodiments of the present disclosure provide a preservative for an in vitro diagnostic reagent. The preservative includes:

a combination of a sulfadoxine solution and a dimethoprim solution, wherein a molar ratio of sulfadoxine in the sulfadoxine solution to the dimethoprim in the dimethoprim solution is from 0.002 to 1.

In view of the above, the embodiments of the present disclosure further provide an aspartate aminotransferase (AST) assay reagent. The AST assay reagent includes the preservative for the in vitro diagnostic reagent as described above.

In view of the above, the embodiments of the present disclosure further provide a triglyceride assay reagent. The triglyceride assay reagent includes the preservative for the in vitro diagnostic reagent as described above.

Compared with the related art, the embodiments of the present disclosure mainly achieve the following beneficial effects:

The in vitro diagnostic reagent preservative according to the present disclosure is almost non-toxic, only inhibits the metabolic link of bacteria, has strong bacteriostatic ability, improves the stability of the reagent. The preservative does not inhibit the compatibility of tool enzymes, does not affect the reaction of the reagent, and can be widely used.

### DETAILED DESCRIPTION

Unless defined otherwise, all technical and scientific terms used herein have the same meaning as commonly understood by one of ordinary skill in the art to which this application pertains. The terms used herein in the specification of present disclosure are only intended to illustrate the specific embodiments of the present disclosure, instead of limiting the present disclosure. The terms "comprise," "include," and any variations thereof in the specification and claims of the present disclosure and in the claims are intended to cover a non-exclusive inclusion. Terms such as "first," "second," and the like in the specifications and claims of the present invention are intended to distinguishing different objects but are not intended to define a specific sequence.

The terms "example" and "embodiment" in this specification signify that the specific characteristic, structures or features described with reference to the embodiments may be covered in at least one embodiment of the present disclosure. This term, when appears in various positions of the description, neither indicates the same embodiment, nor indicates an independent or optional embodiment that is exclusive of the other embodiments. A person skilled in the art would implicitly or explicitly understand that the embodiments described in this specification may be incorporated with other embodiments.

To make a person skilled in the art better understand the technical solutions of the embodiments of the present disclosure, the technical solutions according to the embodiments of the present disclosure are clearly and completely described.

An embodiment of the present disclosure provides a preservative for an in vitro reagent. The preservative includes a combination of a sulfadoxine solution and a dimethoprim solution, wherein a molar ratio of sulfadoxine in the sulfadoxine solution to dimethoprim in the dimethoprim solution is from 0.002 to 1, preferably, from 0.0025 to 1.

In this embodiment, a specific structure of the sulfadoxine is as follows:

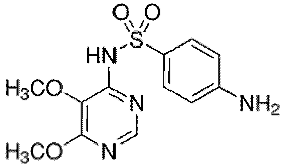

In this embodiment, a specific structure of the dimethoprim is as follows:

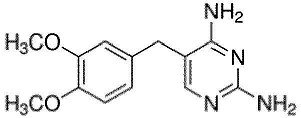

It is understood that folic acid is an essential substance for bacterial growth and also constitutes a folate coenzyme in the body. Under catalysis of dihydropteroate synthase, dihydrofolic acid is synthesized from p-aminobenzoic acid, glutamic acid and dihydropteridine pyrophosphate, or synthsized from p-aminobenzoylglutamic acid and dihydropteridine pyrophosphate, and dihydrofolic acid is then reduced to tetrahydrofolic acid by dihydrofolate reductase. Tetrahydrofolic acid is further synthesized to folate coenzyme f, which provides a carbon unit for the nucleotide synthesis required in bacterial DNA synthesis. The target of sulfadoxine action is bacterial dihydropteroate synthase, which is similar to p-aminobenzoic acid in molecular size and charge distribution. Therefore, sulfadoxine can produce competitive antagonism with p-aminobenzoic acid. The dimethoprim is a reversible inhibitor of dihydrofolate reductase, which hinders the reduction of dihydrofolate to tetrahydrofolate and affects the formation of the coenzyme f, thus affecting the synthesis of microorganisms DNA, RNA and proteins and inhibiting their growth and reproduction. When the sulfadoxine is used in combination with the dimethoprim, a synergistic antibacterial effect is achieved, resulting in double blocking of folic acid metabolism in the bacterial body and an enhancement of the antibacterial effect by several to tens of times.

In this embodiment, the sulfadoxine and the dimethoprim are weighed according to the weight given by a formula amount, and the sulfadoxine is dissolved in a polar organic solvent to prepare a sulfadoxine solution (solution A), and the dimethoprim is dissolved in a polar organic solvent to prepare a dimethoprim solution (solution B), each with a final concentration of 100 mM. The polar organic solvent includes dimethyl sulfoxide, ethanol, methanol, and the like.

In this embodiment, the sulfadoxine solution and the dimethoprim solution are incubated in a warm water bath until the sulfadoxine or the dimethoprim is completely dissolved.

The sulfadoxine solution and the dimethoprim solution are used as stock solutions, and when the sulfadoxine solution and the dimethoprim solution are used as preservatives for in vitro diagnostic reagents, these two stock solutions are diluted and mixed to a predetermined concentration before use.

In this embodiment, a concentration of the sulfadoxine in the in vitro diagnostic reagent is from 0.4 to1500 µM, and a concentration of the dimethoprim in the in vitro diagnostic reagent is from 4 to 1500 µM.

In this embodiment, the concentration of the sulfadoxine in the in vitro diagnostic reagent is preferably from 4 to1000 µM, and the concentration of the dimethoprim in the in vitro diagnostic reagent is preferably from 40 to 1000 µM.

The stock solution is diluted and mixed to a predetermined concentration in the in vitro diagnostic reagent for performing the following experiments.

### Example 1

An AST assay reagent is formulated according to the components and concentrations listed in Table 1.

**Table 1**

| Reagent type | Component | Concentration | pH |
|---|---|---|---|
| R1 | Tris(hydroxymethyl)aminomethane (Tris) | 100 mM | 7.20±0.20 |
| | L-alanine | 500 mM | |
| | Lactate dehydrogenase (LDH) | 3KU/L | |
| | Bovine serum albumin | 2 g/L | |
| | Preservative | / | |
| R2 | Tris(hydroxymethyl)aminomethane (Tris) | 20 mM | 9.20±0.20 |
| | Alpha-ketoglutaric acid | 60 mM | |
| | NADH | 2.0 mM | |
| | Preservative | / | |

In this example, the in vitro diagnostic reagent was an AST assay reagent, the AST assay reagent was a dual reagent, the dual reagent included a first-type reagent R1 and a second-type reagent R2, the dual reagent was stored separately, and when used, the first-type reagent R1 and the second-type reagent R2 were mixed to prepare the AST assay reagent.

The components of AST assay reagent included Tris, L-alanine, LDH, bovine serum albumin, α-ketoglutarate and NADH (Nicotinamide adenine dinucleotide, reduced coenzyme I). The above components were added into purified water respectively according to the concentrations listed in Table 1, mixed well, and the pH value was adjusted.

The sulfadoxine solution and the dimethoprim solutionwere were added as a preservative to the AST assay reagent. Two groups of AST assay reagents were configured as control groups, wherein a combination of the sulfadoxine solution and the dimethoprim solution was added into the first group of AST reagent (i.e., a preservative was added into the first group), and no preservative was added into the second group of AST reagent.

In this example, in the first group of AST reagent, a final concentration of the sulfadoxine is 5 µM and a final concentration of the dimethoprim is 200 µM. Reagent assay parameters: temperature: 37°C, dominant assay wavelength: 340 nm, and secondary assay wavelength: 700nm. 240 µL of R1 was added into 20 µL of samples, and mixed well; upon incubation for 5 min at 37°C, 60 µL of R2 was added and mixed well; upon a delay for 1.5 min, the change rates of absorbance were continuously monitored for 2 to 3 min at the assay wavelength; and ΔA/min values were calculated.

According to the above assay settings, assay was carried out on a Hitachi 7180 automatic biochemical analyzer, the ΔA/min values of 10 clinical samples were recorded, and the results were as listed in Table 2.

**Table 2 Change rate of absorbance of AST test reagent**

| Sample No. | Preservative added ΔA/min | No preservative added ΔA/min |
|---|---|---|
| 1 | 0.0155 | 0.0155 |
| 2 | 0.0393 | 0.0396 |
| 3 | 0.0481 | 0.0487 |
| 4 | 0.0729 | 0.0706 |
| 5 | 0.0308 | 0.0304 |
| 6 | 0.0848 | 0.0829 |
| 7 | 0.0457 | 0.0446 |
| 8 | 0.0332 | 0.0348 |
| 9 | 0.0771 | 0.0756 |
| 10 | 0.0260 | 0.0270 |

As shown by the results in Table 2, there is no significant difference in the change rates of absorbance of the samples assayed by comparison between the samples added with the preservative according to the present disclosure and those not added. This indicates that the preservative according to the present disclosure does not affect the reagent reaction.

### Example 2

A triglyceride (TG) assay reagent is formulated according to the components and concentrations listed in Table 3.

**Table 3**

| Reagent type | Component | Concentration | pH |
|---|---|---|---|
| R1 | piperazine-1,4-bis(ethanesulphonic acid) (PIPES) | 50 mM | 6.80±0.20 |
| | Magnesium chloride hexahydrate | 6 mM | |
| | Adenosine triphosphate | 1.5 mM | |
| | P-chlorophenol | 0.5 mM | |
| | Ethylenediamine tetraacetic acid | 1 mM | |
| | Triton TX-100 | 2 g/L | |
| | Lipoprotein lipase | 4 KU/L | |
| | Glycerophosphate oxidase | 3 KU/L | |
| | Glycerol kinase | 1 KU/L | |
| | Peroxidase | 3 KU/L | |
| | Preservative | / | |

In this example, the in vitro diagnostic reagent was a TG assay reagent, wherein the TG assay reagent is a single reagent. The TG assay reagent included piperazine-1,4-bis(ethanesulphonic acid) (PIPES), magnesium chloride hexahydrate, adenosine triphosphate, p-chlorophenol, ethylenediamine tetraacetic acid, Triton TX-100, lipoprotein lipase, glycerol phosphate oxidase, glycerol kinase, and peroxidase. The above components were added to purified water respectively according to the concentrations listed in Table 3, and mixed well. A pH value was adjusted.

The sulfadoxine solution and the dimethoprim solution were added as a preservative to the TG assay. Two groups of TG assay reagents were configured as control groups, wherein a combination of the sulfadoxine solution and the dimethoprim solution was added into the first group of TG reagent (i.e., a preservative was added into the first group), and no preservative was added into the second group of TG reagent.

In this example, in the first group of TG reagent, a final concentration of the sulfadoxine is 7 µM and a final concentration of the dimethoprim is 400 µM. Reagent assay parameters: temperature: 37°C, dominant assay wavelength: 500 nm, and secondary assay wavelength: 700nm. 200 µL of R1 was added into 2 µL of samples, and mixed well; and upon incubation for 5 min at 37°C, absorbance values A were recorded.

According to the above assay settings, assay was carried out on a Hitachi 7180 automatic biochemical analyzer, the A values of 10 clinical samples were recorded, and the results were as listed in Table 4.

**Table 4**

| Sample No. | Preservative added A value | No preservative added A value |
|---|---|---|
| 1 | 0.0813 | 0.0833 |
| 2 | 0.0651 | 0.0658 |
| 3 | 0.1239 | 0.1262 |
| 4 | 0.0925 | 0.0902 |
| 5 | 0.2933 | 0.2925 |
| 6 | 0.3681 | 0.3564 |
| 7 | 0.1587 | 0.1662 |
| 8 | 0.1876 | 0.1792 |
| 9 | 0.1005 | 0.1006 |
| 10 | 0.0725 | 0.0701 |

As shown by the results in Table 4, there is no significant difference in the absorbance values of the samples assayed by comparison between the samples added with the preservative according to the present disclosure and those not added. This indicates that the preservative according to the present disclosure does not affect the reagent reaction.

### Example 3

A reagent is formulated according to the components and concentrations listed in Table 5.

**Table 5**

| Component | Concentration | pH |
|---|---|---|
| Disodium hydrogen phosphate | 100 mM | 7.20±0.20 |
| Potassium chloride | 75 mM | |
| Sodium chloride | 75 mM | |
| Bovine serum albumin | 2 g/L | |
| Preservative | / | |

In this example, the reagents were provided in three groups. Each group of reagents included disodium hydrogen phosphate, potassium chloride, sodium chloride, and bovine serum albumin, and these components were added into purified water respectively according to the concentrations listed in Table 5, mixed and homogenized, and a pH value was adjusted. Different preservatives were added into the three groups of reagents. No preservative was added into the group A, 0.5 g/L of sodium azide was added as a preservative into the group B, and a preservative containing 6 µM of sulfadoxine and 360 µM of dimethoprim was added into the group C.

These three groups of reagents were stored in an incubator at 37°C for 4 weeks, and finally the bacterial confirmation test was performed for each test material after storage.

Bacterial validation test: small samples of each of the three groups of reagents were smeared and scraped in a culture medium, and placed in an incubator at 37°C for 1 to 5 weeks to visually confirm the presence or absence of bacterial growth, and the results were as listed in Table 6.

**Table 6 Bacterial growth of different groups**

| Group | First week | Second week | Third week | Fourth week | Fifth week |
|---|---|---|---|---|---|
| A | √ | √ | √ | √ | √ |
| B | × | × | × | × | √ |
| C | × | × | × | × | × |
| × indicates no bacterial growth, and √ indicates bacterial growth | | | | | |

As seen from Table 6, bacterial growth is observed within one week in the reagent with no preservative (the group A), bacterial growth was observed as starting at the fifth week in the reagent with sodium azide (the group B), and no bacterial proliferation was observed within 5 weeks in the reagent with the preservative according to the present disclosure (the group C).

### Example 4

A reagent is formulated according to the components and concentrations listed in Table 7.

**Table 7**

| Component | Concentration | pH |
|---|---|---|
| Disodium hydrogen phosphate | 100 mM | 7.20±0.20 |
| Potassium chloride | 75 mM | |
| Sodium chloride | 75 mM | |
| Bovine serum albumin | 2 g/L | |
| Preservative | / | |

In this example, the reagents were provided in 14 groups. Each group of reagents included disodium hydrogen phosphate, potassium chloride, sodium chloride, and bovine serum albumin, and these components were added into purified water respectively according to the concentrations listed in Table 7, mixed and homogenized, and a pH value was adjusted. Combinations of sulfadoxine and µdimethoprim in different amounts were added respectively into these groups.

These 14 groups of reagents were stored in an incubator at 37°C for 4 weeks, and finally the bacterial confirmation test was performed for each test material after storage.

Bacterial validation test: small samples of each of the 14 groups of reagents were smeared and scraped in a culture medium, and placed in an incubator at 37°C for 2 weeks to visually confirm the presence or absence of bacterial growth, and the results were as listed in Table 8.

**Table 8 Bacteriostasis of combination of sulfadoxine and dimethoprim at different concentrations**

| Serial No. | Sulfadoxine (µM) | Dimethoxydine (µM) | Ratio | Bacterial growth |
|---|---|---|---|---|
| 1 | 0 | 0 | - | √ |
| 2 | 0 | 200 | - | √ |
| 3 | 0 | 300 | - | √ |
| 4 | 4 | 0 | - | √ |
| 5 | 8 | 0 | - | √ |
| 6 | 160 | 40 | 4 | √ |
| 7 | 200 | 50 | 4 | √ |
| 8 | 240 | 60 | 4 | √ |
| 9 | 400 | 100 | 4 | √ |
| 10 | 5 | 50 | 0.1 | × |
| 11 | 5 | 500 | 0.01 | × |
| 12 | 20 | 300 | 0.0667 | × |
| 13 | 40 | 400 | 0.1 | × |
| 14 | 60 | 500 | 0.12 | × |
| × indicates no bacterial growth, and √ indicates bacterial growth | | | | |

As seen from Table 8, the addition amount of sulfadoxine and dimethoprim was from 0.002 to 1 in terms of molar ratio, wherein the amount (concentration) of sulfadoxine was from 0.4 to 1500 µM; the amount (concentration) of dimethoprim was from 4 to 1500 µM, within this range, and the inhibitory effect was better.

Based on the combination of sulfadoxine and dimethoprim, the preservative for the in vitro diagnostic reagent according to the present disclosure not only exerts a strong preservative effect as described above, but also is not very toxic like thimerosal and sodium azide, and the diagnostic reagent using the combination as the preservative has excellent stability during storage.

It is apparent that the embodiments described above are only exemplary ones, but not all embodiments of the present disclosure, and these exemplary embodiments do not limit the scope of the present disclosure. The present disclosure may be embodied in many different forms and, on the contrary, these embodiments are provided such that the present disclosure are thoroughly and completely understood. Although the present disclosure has been described in detail with reference to the above embodiments, those skilled in the art will be able to make modifications to the technical solutions disclosed in the specific embodiments or make equivalent substitutions for some of the technical features. Where an equivalent structure made by using the contents of the specification of the present disclosure is directly or indirectly applied to other relevant technical fields, it is likewise within the scope of protection of the present disclosure.

## Claims

1. A preservative for an in vitro diagnostic reagent, comprising:
a combination of a sulfadoxine solution and a dimethoprim solution, wherein a molar ratio of sulfadoxine in the sulfadoxine solution to dimethoprim in the dimethoprim solution is from 0.002 to 1.

2. The preservative according to claim 1, wherein the molar ratio of the sulfadoxine in the sulfadoxine solution to the dimethoprim in the dimethoprim solution is from 0.0025 to 1.

3. The preservative according to claim 1, wherein a concentration of the sulfadoxine in the in vitro diagnostic reagent is from 0.4 to 1500 µM and a concentration of the dimethoprim in the in vitro diagnostic reagent is from 4 to 1500 µM.

4. The preservative according to claim 3, wherein a concentration of the sulfadoxine in the in vitro diagnostic reagent is from 4 to 1000 µM and a concentration of the dimethoprim in the in vitro diagnostic reagent is from 40 to 1000 µM.

5. The preservative according to claim 1, wherein the sulfadoxine solution is prepared by:
weighing sulfadoxine according to a formulated amount, and dissolving the sulfadoxine in a polar organic solvent to prepare the sulfadoxine solution with a concentration of 100 mM.

6. The preservative according to claim 1, wherein the dimethoprim solution is prepared by:
weighing the dimethoprim according to the formula, dissolving the dimethoprim in a polar organic solvent to prepare the dimethoprim solution with a concentration of 100 mM.

7. The preservative according to claim 1, wherein the sulfadoxine solution and the dimethoprim solution are incubated in a warm water bath.

8. An aspartate aminotransferase (AST) assay reagent, comprising the preservative for the in vitro diagnostic reagent of any one of claims 1 to 7.

9. The AST assay reagent according to claim 8, further comprising a first-type reagent R1, a second-type reagent R2, and the preservative for the in vitro diagnostic reagent; wherein the first-type reagent R1 comprises tris(hydroxymethyl)aminomethane, L-alanine, lactate dehydrogenase, and bovine serum albumin; and the second-type reagent R2 comprises tris(hydroxymethyl)aminomethane, alpha-ketoglutarate, and NADH.

10. The AST assay reagent according to claim 9, wherein in the first-type reagent R1, a concentration of the tris(hydroxymethyl)aminomethane is 100 mM, a concentration of the L-alanine is 500 mM, a concentration of the lactate dehydrogenase is 3 KU/L and a concentration of the bovine serum albumin is 2 g/L.

11. The AST assay reagent according to claim 9, wherein in the second-type reagent R2, a concentration of the tris(hydroxymethyl)aminomethane is 20 mM, a concentration of the alpha-ketoglutarate is 60 mM, and a concentration of the NADH is 2.0 mM.

12. The AST assay reagent according to claim 9, wherein the AST assay reagent is configured as two control groups; wherein the preservative for the in vitro diagnostic reagent is added into one control group, and the preservative for the in vitro diagnostic reagent is not added into the other group.

13. The AST assay reagent according to claim 12, wherein the AST assay reagent with the preservative has a final concentration of the sulfadoxine of 5 µM and a final concentration of the dimethoprim of 200 µM.

14. A triglyceride assay reagent, comprising the preservative for the in vitro diagnostic reagent of any one of claims 1 to 7.

15. The triglyceride assay reagent according to claim 14, further comprising piperazine-1,4-bis(ethanesulphonic acid), magnesium chloride hexahydrate, adenosine triphosphate, p-chlorophenol, ethylenediamine tetraacetic acid, Triton TX-100, lipoprotein lipase, glycerol phosphate oxidase, glycerol kinase, and peroxidase.

16. The triglyceride assay reagent according to claim 15, wherein a concentration of the piperazine-1,4-bis(ethanesulphonic acid) is 50 mM, a concentration of the magnesium chloride hexahydrate is 6 mM, a concentration of the adenosine triphosphate is 1.5 mM, a concentration of the p-chlorophenol is 0.5 mM, a concentration of the ethylenediamine tetraacetic acid is 1 mM, a concentration of the magnesium chloride hexahydrate is 6 mM, a concentration of the magnesium chloride hexahydrate is 6 mM, a concentration of the Triton TX-100 is 2 g/L, a concentration of the lipoprotein lipase is 4 KU/L, a concentration of the glycerol phosphate oxidase is 3 KU/L, a concentration of the glycerol kinase is 1 KU/L, and a concentration of the peroxidase is 3 KU/L.

17. The triglyceride assay reagent according to claim 15, wherein the triglyceride assay reagent is configured as two control groups; wherein the preservative for the in vitro diagnostic reagent is added into one control group, and the preservative for the in vitro diagnostic reagent is not added into the other group.

18. The triglyceride assay reagent according to claim 17, wherein the triglyceride assay reagent with the preservative has a final concentration of the sulfadoxine of 7 µM and a final concentration of the dimethoprim of 400 µM.
